# EUROPEAN PATENT APPLICATION

(11) **EP 3 892 259 A1**
(43) Date of publication of application: **13.10.2021**
(21) Application number: 20168966.8
(22) Date of filing: 09.04.2020
(51) Int. Cl.: A61K 9/00, A61L 27/14, A61L 27/54, A61L 27/56, A61L 27/58

(54) **MEMBRANES FOR MEDICAL DEVICES**

(71) Applicant: Defymed, 67200 Strasbourg (FR)
(72) Inventor: BURCEZ, Charles-Thibault, 67200 STRASBOURG (FR); BOU AOUN, Richard, 67200 STRASBOURG (FR); SIGRIST, Séverine, 67200 STRASBOURG (FR)
(74) Representative: Flesselles, Bruno F.G.

(57) **Abstract**

The invention relates to an implantable chamber comprising a closed shell made of a permeable membrane, said membrane comprising at least one layer of porous biocompatible polymer, with pores controlled and homogeneously distributed over the membrane.

## Description

The invention relates to the field of medical devices, for administration of medicinal substances, which are implantable and in particular which are in the form of pouches, closed shells or chambers and can be used for diffusing a substance of interest within a subject's body. The membranes which enable such chambers and devices to be manufactured are also subjects of the invention.

The treatment of pathological conditions requiring a continuous supply, to the body, of substances of therapeutic interest has made necessary the development of devices which can be implanted in a patient and are capable of releasing these substances efficiently and sometimes for long periods of time.

Implantable chambers (or pouches) have been described for administering substances of interest *in situ* to subjects in need thereof. In particular,

Examples of devices (bioartificial organs, semi-permeable membranes, encapsulating chambers) are known in the prior art.

Mention may thus be made of WO 02/060409 which describes a membrane consisting of a porous polycarbonate biocompatible polymer which is surface-modified by generation of polar sites and covered with a layer of at least one hydrophilic polymer, and the use thereof for manufacturing bioartificial organs.

WO 2012/017337, US 2013/131828 and FR 2960783 describe a functionalized semi-permeable membrane composed of a porous biocompatible support pretreated so as to increase the surface energy thereof and comprising at least two layers, each comprising a hydrophilic polymer and at least one biologically active molecule, and also the use thereof in particular for manufacturing a bioartificial organ and an encapsulation chamber. The biologically active molecules disclosed in this application are VEGF and heparin, which is, in particular, present in a HPMC or EC layer.

WO 2012/010767 describes a device for forming an implantable artificial organ, which comprises a closed shell made of a semi-permeable membrane.

WO 2000/060051 describes an encapsulation chamber, the semi-permeable membranes of which can be made from different materials and polymers (see page 21, line 15 to page 22, line 23 of this document).

WO 2015/086550 describes a chamber for encapsulating secreting cells producing at least one substance of therapeutic interest, comprising a closed shell made of a semi-permeable membrane, delimiting a space capable of containing said secreting cells producing at least one substance of therapeutic interest, wherein said membrane comprises at least one layer of porous biocompatible polymer, and one layer of non-woven biocompatible polymer.

WO 2006/080009 discloses an implantable device chamber for encapsulating secreting cells, the chamber(s) being encapsulated by a membrane made of non-woven electrospun fibers and being further impregnated with heparin.

WO 2018/087102 discloses a kit comprising a) a chamber (pouch) comprising a closed shell made of the semi-permeable membrane, comprising at least one connector comprising a body attached to the sheet, and a conduit connected to the connector so as to be in hydraulic communication with the inside of the pouch and a catheter that can be connected to said connector at one end and that can be connected to a source of delivery of a compound of interest at the other end. This pouch is implanted within the subject's body for *in situ* delivery of compounds of interest by diffusion through the pores of the semi-permeable membrane, the compounds reaching the inside of the pouch through the catheter.

US 20070016171 describes an implantable device for administering a liquid, in particular a medication, in man or animal, comprising a transfer chamber, transfer means for transferring liquid between the transfer chamber and a supply enabling the liquid to be delivered, the supply including an inlet for receiving the liquid and at least one outlet for delivering the liquid to the patient. Such device

All the above devices need to resist tearing strengths that exist when the device in implanted in the subject's body. Consequently, the membranes described in these documents have mechanical properties with good tensile resistance force at break but very low stretching and elongation forces.

It is thus not possible to fold these membranes, which hampers the ability to use mini-invasive surgery for implantation. Indeed, it is impossible to roll the device (the chamber) to pass through a trocar, without damaging the membranes. Indeed, the presence of the pores in the membrane (needed to allow diffusion of the substance of interest) is an element that can weaken the membranes when these are bent or when cracks, tears appear between pores.

It is however of interest to be able to perform such mini-invasive surgery (rather than open surgery), as it would reduce pain, lessen scarring, reduce wound complications, reduce patient recovery time, reduce patient stay at the hospital, increase patient satisfaction. It would also allow to implant the device at other implantation sites than the ones available upon open surgery.

It is thus needed to provide new chambers that would have maintained tensile resistance force at break (i.e. no disruption when implanted), and improved elongation properties. In particular, these chambers should be bent or folded (in particular to be used though mini-invasive surgery with a trocar) and go back to their original form when released from the trocar and after implantation. Surprisingly, the Applicant has shown that the problems observed with the membranes of the prior art can be solved when the pores are evenly (or homogeneously, or uniformly) distributed at the surface of the membranes. Indeed, the pores of the prior art are made by track etching, which leads to a random distribution and non-controlled setting of the pores. These chambers are to be used to diffuse a substance of interest within a subject's body and can also be termed "diffusion chambers".

Consequently, the invention pertains to a biocompatible implantable chamber, comprising a closed shell made of a membrane which delimits an inner space, wherein the membrane comprises pores that are present on at least one part of its surface, and wherein the pores are homogeneously distributed on at least one part of the membrane.

It is recalled that the term "biocompatible" is said of a material which is well tolerated by a living organism and which does not cause, by itself, a rejection reaction, a toxic reaction, a lesion or a harmful effect on the biological functions of the latter. One can't exclude the possibility of an inflammatory reaction due to the insertion of the material into the organism.

By "homogeneously distributed", it is intended to indicate that pore density on the surface of the membrane (where they are present) is essentially constant and equal to the mean density +/- 10 %. Such "local" pore density (as opposed to the mean pore density) is measured on a portion of the whole surface of the membrane, generally about 1 cm², more preferably about 0.5 cm², more preferably about 0.1 cm², preferably about 0.05 cm² or 0.01 cm². In this case, it is interesting to measure pore density on multiple "local" parts of the membrane. It is also to be noted that the pores can be distributed only on a portion of the membrane surface. In this case, the homogeneous distribution (local and mean densities) is to be assessed on the surface where the pores are present.

In a preferred embodiment, the membrane comprises more than one a layer of biocompatible porous polymer. Such multilayered membranes are described and known in the art. In particular, the membrane may contain a layer of non-woven between two layers of porous polymers. Such designs are described in WO 2015/086550. In this embodiment, it is preferred when the pores are homogeneously distributed at the surface each of the two layers of porous polymers.

It is however preferred when the membrane consists in one layer of porous biocompatible polymer, wherein the pores are homogeneously distributed on the membrane.

### Description of the membrane forming the chamber

### Porous biocompatible polymer

A porous biocompatible polymer is known in the art. It may be chosen from polycarbonate (PC), polyurethane (PU), polyethylenimine, polyolefins (in particular polypropylene (PP) or polyethylene (PE)), polyester (in particular poly(ethylene terephthalate) (PET)), poly(vinyl chloride) (PVC), fluoropolymers (in particular polytetrafluoroethylene (PTFE) or polyvinylidene fluoride (PVDF)), and polyamides. Such polymers can be obtained by any method known in the art.

In one particular embodiment, at least one layer of the membrane is made of poly(ethylene terephthalate) (PET).

The use of a porous biopolymer in the chamber's membrane will make the chamber permeable. According to the invention, pores are formed within the film of biocompatible polymer so as to make it porous, and in such a way that the pores are homogeneously distributed. It is also to be noted that the membrane is flexible enough to be folded/rolled so that it can be implanted by minimally invasive surgery.

It is thus important to use a controlled method to perforate the layer of biocompatible polymer, to control the localization and distribution of the pores and make sure that the distribution is homogeneous and uniform. Furthermore, it is also interesting to have standardized pored (i.e. pores that have essentially the same diameter and are essentially all identical).

The pore formation in the prior art is described as being performed by the electron bombardment method or the heavy ion bombardment method (this second technique is in particular described in patent US 4 956 219). In the case of heavy ion bombardment, the density of the heavy ions bombarded at the surface of the biocompatible support determines pore density, while the chemical erosion treatment time determines the pore size. The porous membranes of the prior art are thus prepared using the "track-etching" process known in the prior art and described in particular in patents US 4 956 219, DE19536033 or CH701975. This technology, used for the membranes of the prior art, consists in irradiating a polymer film by means of energetic heavy ions, resulting in the formation of linear latent traces characterized by a local degradation of this polymer; these traces are then revealed in the form of pores by means of a selective chemical attack. The membrane is beamed with heavy ions. The heavy ions pass through the entire thickness of the polymer film. In passing through the polymer, the heavy ions destroy or cut the polymer chains and thus form a clean straight opening through the material. The final alignment of the pores is determined by the angle of the beam relative to the polymer film during the irradiation process. The beam may thus be perpendicular to the polymer film or at any other angle. In the next step, the film is passed through a bath of a strong acid such as nitric acid and the openings become pores after contact with alkaline solutions such as sodium hydroxide or potassium hydroxide. Contrary to the rest of the film, these openings made by the ions allow the alkaline solution to pass through, said alkaline solution filling them and allowing the etching of the pores by removing the material (polymer) around these openings. The pore size is controlled by the concentration of the alkaline solution, the contact time and the temperature of the solution.

Although this technology enables the production of porous polymer membranes characterized in particular by a flat surface and a narrow cut-off threshold, it is not controlled, as it is not possible to control the electron or ion beam. Consequently, the distribution of the pores is not homogeneous and uniform (some parts of the membrane surface contain a higher pore density than other parts). Furthermore, the size (diameter) of the pores is not well controlled. It is indeed possible that two ions land on the membrane close to each other, thus leading to a "double" pore (a pore that is made by both ions). Finally, this method leads to some variability in the shape of the pore, that depend on the angle with which the ions touch the surface of the polymer, such angle may vary within the bean.

Consequently, a method which allows control of the number, density, diameter and shape of the pores is used to prepare the membranes herein disclosed.

It is preferred to use the laser technology, described in Caiazzo et al (Journal of Materials Processing Technology 159 (2005) 279-285) or Badoniya (International Research Journal of Engineering and Technology (IRJET), Volume: 05 Issue: 06 | June -2018, CO2 Laser Cutting of Different Materials).

The laser cutting process consists to focus precisely a laser beam through the material to cut.

By focusing the large beam down to a single pinpoint, the heat density at that spot is extreme. Focusing the laser beam can be done by a special lens, or by a curved mirror, and this takes place in the laser cutting head. The high-power density results in rapid heating, melting and partial or complete vaporizing of the material.

The beam intensity, length and heat output depending on the material as well as the use of mirrors or special lens to further focus the laser beam makes the laser cutting a very well-controlled process. Moreover, using laser cutting complex shapes can be achieved.

Depending on the technology used (continuous wave beam or pulsed beam), the mechanism of perforating the material varies.

It is recalled that continuous wave beam is obtained by applying constantly an energy source to the laser medium, whereas pulsed beam is obtained by using a discontinuous pump source, leading to short, intense and discontinuous laser beam pulses.

The laser cutting technique makes it possible to obtain controlled generation of pores, with an accurate and smooth finish

CO₂ cutting is most often used on non-metal materials as they have a wavelength of 10.6 micrometers (lower energy than). Therefore, CO₂ lasers are preferred to cut thin plastic films whereas Nd:YAG lasers (YAG (yttrium aluminum garnet) lasers doped with Neodymium) having wavelength, around 1.064 micrometers can be used for both metals and non-metals materials.

The pores are preferentially cylindrical, but this technology can also make it possible to obtain pores of other shape, such as of conical shape.

Preferentially, the pores are aligned.

This technology is applicable to various materials, such as the ones cited above.

It is possible to obtain pores with a controlled size of between 200 nm and 100 µm, a pore density of between 10³ pores/cm² and 10⁹ pores/cm², to use membranes with a thickness of between 5 µm and 200 µm.

It is to be noted that, without the treatment to form pores on the biocompatible polymer, such layer of biocompatible polymer would remain impervious to any substance, and would not allow diffusion of the substance of interest from the inner part of the biocompatible organ to the outer part. The pores only allow the diffusion of substances that are below the cutoff (*i.e.* that are smaller than the pore diameter). In this specific use, the most important is to avoid platelets aggregation within the chamber, and the cut-off will thus be chosen as to forbid entry of platelets in the chamber.

It is thus clear that, when the membrane contains a non-woven layer, such non-woven layer and such layer of the porous biocompatible polymer are different layers, made of different materials, and presenting different properties (in particular with regards to the passing and diffusion of substances through each layer).

### Non-woven polymer

The membrane may comprise a layer of a non-woven polymer (non-woven). Such polymer is such that the fibers thereof are maintained randomly. It is thus a sheet consisting of fibers oriented in a particular direction or randomly, bonded by friction and/or cohesion and/or adhesion. The fibers are thus arranged statistically, i.e. deposited randomly. Consequently, and due to the random arrangement of the fibers, the non-woven polymer is permeable to substances, and there is no control of the size of the substances that can diffuse within the non-woven polymer. A non-woven membrane is not structured as the porous membranes and there is no control of diffusion of substance within the non-woven part of the membrane, in contrast to porous membranes, which present a cut-off, depending on the size of pores, as described below.

Non-woven polymers can be produced using polymeric fibers of any type. Mention may thus be made of polyesters: PET (poly(ethylene terephthalate)), PBT (poly(butylene terephthalate)), PVC (poly(vinyl chloride)), PP (polypropylene), PE (polyethylene) or blends of these polymers. Polyamides or polycarbonates can also be used to produce non-woven polymers. Preferably, the non-woven polymer is chosen from polycarbonate (PC), polyester, polyethyleneimine, polypropylene (PP), poly(ethylene terephthalate) (PET), poly(vinyl chloride) (PVC), polyamide and polyethylene (PE). Blends of these polymers can also be used for producing the non-woven polymer. Poly(ethylene terephthalate) (PET) is particularly preferred.

Generally, this non-woven polymer is obtained by the meltblown method. The composition thereof is an entanglement of microfibers which have been "melt blown". The fibers have been obtained by any method known in the art, in particular the polymers herein used can be obtained by any method known in the art, in particular electrospinning, which avoids use of solvents, which is advantageous for the clinical application of the device and kit. This method of production is particularly suitable for polymers which can be melt spun, in particular polypropylene, poly(ethylene terephthalate), polyamides or polyethylene. This method generates non-wovens of greater mechanical strength. Usable materials are disclosed in particular in WO2015086550, PCT/EP2016/061051, EP1357896 or WO2012010767.

When the membrane comprises multiple layers of polymers, their combination is preferentially carried out by lamination, using methods known in the art, such as thermal lamination, with or without the presence of adhesives, preferably without adhesive.

### Physical characteristics of the biocompatible membrane

### Pore density:

It is preferred when the pore density is greater than 10³ pores/cm², preferably greater than 10⁴ pores/cm². This pore density is generally less than 10⁹ pores/cm², preferably less than 10⁷ pores/cm².

Use is therefore made of membranes which can have a pore density preferentially greater than 10³ pores/cm², more preferably greater than 10⁴ pores/cm². This density is preferentially less than 10⁹ pores/cm², or even less than 10⁷ pores/cm², or less than 10⁶ pores/cm² This density is therefore between 10³ pores/cm² and 10⁷ pores/cm², preferably between 10⁴ pores/cm² and 10⁶ pores/cm². A density greater than 10⁴ and less than 10⁵ pores/cm² is perfectly suitable.

Consequently, the density of the pores on the implantable chamber is between 10³ pores/cm² and 10⁹ pores/cm², preferably between 10⁴ pores/cm² and 10⁷ pores/cm², more preferably between 10⁴ pores/cm² and 10⁶ pores/cm².

### Pore diameter

As seen above, the pores of the porous biocompatible polymer have an internal diameter such that they allow permeability of the membrane, so that the substance of interest can diffuse through the membrane's pores.

Thus, the diameter of the pores of the membrane is generally comprised between 200 nm and 100 µm, preferably between 200 nm and 50 µm, or between 1 µm and 50 µm, more preferably between 5 µm and 50 µm.

### Membrane thickness

The membrane has a thickness between 5 µm and 250 µm, preferably between 5 µm and 150 µm, preferably between 10 µm and 150 µm. Thus the thickness is greater than 5 µm. it is generally less than 250 µm, in particular less than 200 µm, preferably less than 150 µm. A thickness of about 35 µm to about 125 µm is perfectly suitable.

### Description of the chamber

The invention uses a chamber for allowing *in situ* diffusion of at least one compound or substance of (therapeutic) interest. This chamber comprises a closed shell made of a membrane as described herein, delimiting a volume in which the compound of interest will transit from the catheter, and before *in situ* diffusion.

This chamber can also be referred to as a "pouch" or a "diffusion chamber". In one embodiment, the exterior (outer part) and interior (internal part) of the chamber is functionalized with a molecule having biological activity, such as heparin.

Such chambers are described in application WO 2012/010767. In one preferred embodiment, the shell is formed from two membranes as disclosed herein, which are heat-welded together. Use may be made of the method described in WO 2012/010767 or a method of heat-welding using ultrasound, known in the art. Membranes can also be sealed on their periphery, to form the chamber, using any thermosealing method known in the art.

### Shape of the chamber

In one preferred embodiment, the chamber is circular. Such a shape has several advantages:
- absence of "corners" or protruding parts which are capable of creating cell or inflammatory aggregates during the implantation,
- ease of manufacture of the chamber In one particular embodiment, the diameter of the chamber is greater than 1 cm. It is generally less than 20 cm, and is preferentially less than 15 cm, or than 12 cm. A diameter of between 2 and 12 cm is perfectly acceptable.
- When the chamber is not round, the largest dimension thereof is generally greater than 2 cm. It is generally less than 20 cm, and is preferentially less than 15 cm, or than 12 cm. A largest dimension of between 2 and 12 cm is also perfectly acceptable.

### Volume of the chamber

As seen above, the chamber delimits a volume into which the solution containing the compound of interest will transit before diffusion at the exterior of the chamber, through the pores of the membrane forming the envelope of the chamber. The volume of the chamber is generally comprised between 500 µl and 20 ml and depends on the dimensions of the diffusion chamber.

It is also preferred when a rigid part is added to the chamber. Such rigid part can be a connector as disclosed below, either located within a membrane, or within the weld between the membranes. Such rigid part is useful to allow gripping of the chamber by pliers when microsurgery (laparoscopy surgery) is used for implanting the device. The rigid part may also be useful to roll the chamber around a trocar for such surgery.

### Other polymers

It is possible to add various layers of polymers (such as any hydrophilic polymer) to the surface of the membrane, and in particular on the surface of the porous polymer. These layers of polymer can be added by using the Layer-by-Layer deposition method. Examples are provided in WO 2012/017337.

A hydrophilic polymer is a polymer or a blend of polymers, which, after application on a film of porous biocompatible polymer, has an angle value of less than 40°, preferably less than 30°, after measurement according to the "sessile drop" test described in Example 2 of WO 02/060409.

It should be noted that the angle value according to the "sessile drop" test can vary depending on the treatment of the polymer. Thus, contact angles of less than 20° (of about 16-17°) can be observed for the biocompatible biopolymer, when two plasma treatments are carried out, this angle increasing (generally less than 30°) when the hydrophilic polymer (in particular HPMC) is deposited after the two plasma treatments. If a blend of hydrophilic polymers, which also contains a molecule with biological activity (in particular an HPMC, ethylcellulose + heparin mixture), is used, the angle may be greater than 30°, but remains less than 40°.

Preferentially, the hydrophilic polymer is soluble in water. This is because, due to of the implantation of the bioartificial organ in the body of a host organism, use of organic solvents is excluded since their complete elimination is difficult, and their presence, even in small amounts, is not compatible with a therapeutic or surgical use in humans or animals.

Preferably, the hydrophilic polymer material is chosen from the following hydrophilic polymers:
- celluloses and derivatives thereof, such as ethylcellulose (EC), hydroxypropylmethylcellulose (HPMC) or carboxymethylcellulose (CMC);
- polyacrylamides and copolymers thereof;
- polyvinylpyrrolidone (PVP) and copolymers thereof;
- polyvinyl alcohols;
- vinyl acetate copolymers, such as a poly(vinyl acetate)/poly(vinyl alcohol) copolymer;
- polyethylene glycols;
- propylene glycols;
- hydrophilic poly(meth)acrylates;
- polysaccharides;
- chitosans.

As hydrophilic polymer, use is made of both a polymer material consisting of one of the hydrophilic polymers as defined above and a blend of several of the hydrophilic polymers above, generally a blend of two or three of the hydrophilic polymers above.

Preferably, the hydrophilic polymer is chosen from cellulose-based compounds, in particular HPMC, EC, TEC or CMC, polyvinylpyrrolidones, poly(vinyl alcohol)s, or polyacrylates such as poly(hydroxyethyl acrylate) (HEA) or acrylic acid copolymers.

The hydrophilic polymer may also be composed of a blend of two or more hydrophilic polymers mentioned above, in particular a blend of HPMC and CMC, or of HPMC and EC.

Celluloses and cellulose derivatives, in particular hydroxypropylmethylcellulose (HPMC), are preferred.

### Addition of an active molecule at the surface of the membrane

The membrane may be functionalized with an active molecule, such as heparin or another molecule as disclosed below.

One should note that it is possible to functionalize (such as bridging heparin) the membranes before forming the chamber. In this case, as indicated elsewhere, the heparin should be, at least, on the outer surface of the chamber.

Alternatively, it is possible to form the chamber and then functionalize it, for instance by dipping it in various baths containing a priming solution, and the solution containing the functionalizing molecule.

It is possible that a hydrophilic polymer deposited on the layer of porous biocompatible polymer can optionally contain an active molecule.

This "active molecule" is mixed with the hydrophilic polymer. It is intended to be released into the medium surrounding the semi-permeable membrane in particular in order to reduce the inflammation due to the implantation of the bioartificial organ, and/or to induce a positive response (in particular increased vascularization) by the tissue(s) of the patient receiving the bioartificial organ.

Thus, the active molecule is chosen from anti-inflammatory agents, anti-infective agents, anaesthetics, growth factors, agents which stimulate angiogenesis and/or which induce vascularization, agents which induce healing, immunosuppressive agents, antithrombotics including antiaggregants and anticoagulants, angiotensin-converting enzyme (ACE) inhibitors, or any molecule which stimulates insulin secretion (IGF, glucagon-like peptide 1 (GLP-1) or its derivatives, incretin mimetics).

Among the anti-inflammatory agents, mention may be made of nonsteroidal anti-inflammatories (NSAIDs), such as acetaminophen, aminosalicylic acid, aspirin, celecoxib, choline magnesium trisalicylate, declofenac, diflunisal, etodolac, flurbiprofen, ibuprofen, indomethacin, interleukin IL-10, IL-6 mutein, anti-IL-6, NO synthase inhibitors (for example, L-NAME or L-NMDA), interferon, ketoprofen, ketorolac, leflunomide, mefenamic acid, mycophenolic acid, mizoribine, nabumetone, naproxen, oxaprozin, pyroxicam, rofecoxib, salsalate, sulindac and tolmetin, and corticoids such as cortisone, hydrocortisone, methylprednisolone, prednisone, prednisolone, betamethasone, betamethasone dipropionate, betamethasone valerate, beclomethasone dipropionate, budesonide, dexamethasone sodium phosphate, flunisolide, fluticasone propionate, paclitaxel, tacrolimus, tranilast, triamcinolone acetonide, fluocinolone acetonide, fluocinonide, desonide, desoximetasone, fluocinolone, triamcinolone, clobetasol propionate and dexamethasone. Ibuprofen is particularly suitable and preferred.

Use is preferably made of antithrombotics such as antiaggregants (acetylsalicylic acid, clopidogrel, ticlopidine, dipyridamole, abciximab, eptifibatide and tirofiban), anticoagulants (heparin, bivalirudin, dabigatran, lepirudin, fondaparinux, rivaroxaban, epoprostenol, warfarin, phenprocoumon, protein C, drotrecogin alfa, antithrombin, pentosan) and thrombolytics (alteplase, urokinase, tenecteplase and reteplase).

The use of a heparin is particularly preferred.

In another embodiment, ibuprofen is used.

In addition, it is possible to use a molecule which makes it possible to induce vascularization of the tissues surrounding the bioartificial organ, in particular PDGF (platelet derived growth factor), BMP (bone morphogenetic protein), VEGF (vascular endothelial growth factor), VPF (vascular permeability factor), EGF (epidermal growth factor), TGF (transforming growth factor) and FGF (fibroblast growth factor).

It is also possible to use IGF-1 and IGF-2, a neurotrophic factor (NGF).

In one particular embodiment, a cell growth factor is chosen which promotes vascularization by inducing angiogenesis, such as basic fibroblast growth factor (bFGF), vascular endothelial growth factor (VEGF), platelet derived endothelial cell growth factor (PDGF A or B), bone morphogenetic protein (BMP 2 or 4), or hepatocyte growth factor (HGF).

One can also use antimicrobial molecules, or any useful molecule such as polypeptides (in particular antimicrobial peptides), ionized silver, copper, zinc, non-metal elements such as hydrogen, chlorine, iodine, or oxygen, selenium.

For the preparation of the layer of hydrophilic polymer and biologically active molecule, the hydrophilic polymer or the blend of hydrophilic polymers is dissolved in water.

The addition of the hydrophilic polymer optionally containing an active molecule to the layer of porous biocompatible polymer is carried out according to the methods described in WO 02/060409 and WO 2012/017337.

In another embodiment, it is possible to add, at the surface of the porous biocompatible polymer, two layers each comprising a hydrophilic polymer and at least one biologically active molecule, as described in WO 2012/017337.

Reference is further made to EP86186, EP1112097, EP1112098, EP658112 and WO2012017337.

There are other ways to bind an active molecule to the surface of the membrane, illustrated with heparin below.

### Binding Heparin to a straight-chained organic polymer

In a specific embodiment, said heparin layer consists in a substantially straight-chained organic polymer having a number of functional groups distributed along the polymer backbone chain, via which groups at least 20 molecules of heparins are anchored through covalent bonds, wherein the heparins are bound to the polymer backbone chain *via* an amino group or amino acid associated with the heparins, and wherein said heparin layer is affinity bound to the surface of said layer of porous biocompatible polymer.

This method is actually disclosed in EP 658112.

Briefly, one uses an at least substantially water-soluble, biologically active conjugate (macromolecule), preferably in substantially pure form, comprising a substantially straight-chained organic homo- or heteropolymer having a number of functional groups distributed along the polymer backbone chain, via which groups of at least about 20 molecules of heparin are anchored through covalent bonds wherein heparin is bound to the polymer backbone chain via an amino group or amino acid associated with heparin. It could be noted that heparan sulphate, dermatan sulphate, chondroitin sulphate, but also fragments and derivatives of these substances which are functional for the purpose can be equivalently used and substituted to heparin. As indicated in EP 658112, the number of heparin residues per polymer backbone chain is, as mentioned above, at least 20, but preferably higher, usually at least 30. Depending on the polymer backbone chain used, it may be preferred to have at least 60 and even more than 100 heparin residues per polymer backbone chain. The upper limit depends on the circumstances and is set *inter alia* by the solubility properties of the selected carrier polymer, how high a viscosity that may be permitted, and the like.

### The substantially linear polymer

The substantially linear polymer chain is preferably substantially biologically inert after the coupling of heparin, i.e. devoid of interfering biological activity.

It should present a number of functional groups, such as amino, hydroxyl or carboxyl groups, distributed along the chain and capable of, after optional modification, being coupled to the heparin, either directly or via a coupling sequence.

The carrier polymer should preferably have a good solubility in water. At least it should, in accordance with what has previously been said about the conjugate, be at least substantially water-soluble after the coupling of heparin.

Specific and preferred polymer chains are natural or synthetic polypeptide, polysaccharide or aliphatic polymer, such as polylysine, polyornithine, chitosan, polyimine and polyallylamine.

### Binding of the conjugate polymer / heparin to the membrane surface

This conjugate is bound to the membrane surface, which may have been prepared to have affinity to the conjugate (usually but not necessarily to the heparin residues) so as to thereby provide the surface with the desired biological activity.

The functionalized membrane is obtained by simply contacting, under suitable conditions, the conjugate (comprising the organic polymer having a number of functional groups, via heparin molecules anchored by covalent bonds), with the membrane surface prepared to present affinity to the conjugate.

A preferred form of affinity between the conjugate and the substrate surface is of electrostatic nature, and more particularly that binding takes place by electrostatic interaction between the heparin residues and the membrane surface. Indeed, one will use the electrostatic net charge of the conjugate, which is sufficient to permit substantially irreversible binding to an oppositely charged membrane surface. Since the conjugate is negatively charged, the membrane surface shall be or shall be made cationic.

Various methods for making the membrane surface cationic are well known. Treatment with polyimine is a suitable method, but also other polyamines, such as polylysine, chitosan or polyallylamine, may be used. These polymers are used in the examples of EP 658112.

### Coupling of heparin to the polymer

There are different ways to couple the heparin to the substantially straight-chained organic polymer.

It is however preferred that each heparin molecule is bound terminally and by only a single bond to the carrier polymer. Suitably, the heparin molecule is bound via an amino acid, and then preferably a terminal amino acid, but also free amino groups of a heparin unit may be used. The latter may exist free as such or may have been liberated through desulphation or deacetylation.

In particular, the heparin may be bound directly to an amino-functional polymer chain utilizing a nitrous acid degraded heparin having a terminally located aldehyde group prepared according to the method described in US 4,613,665.

Preferably, the heparin is bound to the polymer chain by means of a coupling reagent, and preferably a heterobifunctional one, such as N-succinimidyl-3-(2-pyridyldithio)-propionate (SPDP).

As a way of illustration, coupling of heparin to a polylysine (having a molecular weight above 400,000) will be described to lead a conjugate having up to 500 heparin chains per carrier molecule may be prepared.

N-succinimidyl-3-(2-pyridyldithio)-propionate (SPDP), is coupled to amino groups on the polylysine, the SPDP-substituted polylysine then being purified chromatographically. In a separate coupling step, SPDP is also coupled to amino groups on heparin which are present either in terminal amino acid residues or as free glucosamine (the latter content may be controlled via N-desulphation or N-deacetylation). The SPDP-groups are reduced to thiol function, whereupon the SH-substituted heparin is purified chromatographically. The content of SPDP groups in polylysine and SH-groups in heparin, respectively, are determined spectrophotometrically, and heparin is mixed with polylysine in an equimolecular amount with regard to SPDP and SH, heparin being bound covalently to polylysine via disulphide exchange, the reaction rate of which may be followed spectrophotometrically. The precipitation reaction between polylysine and heparin does not take place when polylysine has been provided with SPDP-groups, even if only a certain portion of the amino groups of polylysine have been substituted. Practical experiments have shown that the disulphide exchange is quicker and proceeds to completion only at a high salt concentration (suitably 3 M NaCI). When the reaction is completed, the conjugate is purified chromatographically, free heparin and low-molecular reaction products being removed.

### Binding heparin by end-point attachment to the layer

In another embodiment, said heparin layer consists in heparin molecules covalently bound to a layer of a polymer applied on the surface of said layer of porous biocompatible polymer.

Methods to perform such embodiment are disclosed in details in EP 86186, EP 1112097 and EP 1112098.

The membrane surface is primed by applying a polymeric base matrix using a layer-by-layer technique. As a matter of illustration, the preferred method is to use a cationic amino polymer to be adsorbed to the material surface, followed by an anionic polymer and a polymeric amine. Additional layers of anionic and cationic polymers may also be applied to achieve optimal functional characteristics and coverage of the underlying material.

The technology is based on a chemical modification of heparin (diazotization, usually performed in an aqueous solution with a suitable diazotizing agent, e.g. a nitrite, such as sodium nitrite, in acid solution or butyl nitrite) that results in formation of a reactive aldehyde group at one end of the linear molecule. These groups are then reacted with primary amino groups incorporated on the material surface by the priming procedure, leading to formation of Schiff's bases, which are then reduced with a suitable reducing agent, such as a cyanoborohydride, preferably of an alkali metal, such as sodium, potassium or lithium, to yield stable covalent bonds.

### Bioartificial organ

In a specific embodiment, the substance is secreted by cells present in the chamber (which can thus be termed a bioartificial organ). In some embodiments, the size of the pores may allow cells and/or molecules of the immune system to pass through the pores and potentially interact with cells present in the chamber. Consequently, in such embodiments, such cells present in the chamber are preferably encapsulated, by methods known in the art, unless such cells are not immunogenic (such as Autologous cells). As a matter of illustration, one can cite microencapsulation, such as the systems described in Calafiore (J Diabetes Investig. 2018 Mar;9(2):231-233), Espona-Noguera et al (Pharmaceutics 2019, 11, 597),

In this case, the chamber may contain connectors linked to a catheter (see below) to allow flushing and replacing the cells.

### Kit

The invention also pertains to a kit comprising
a. an implantable chamber as disclosed above, which comprises at least one connector comprising a body attached to the membrane, and a conduit connected to the connector so as to be in hydraulic communication with the inside of the pouch
b. a catheter that can be connected to said connector at one end and that can be connected to a source of delivery of a compound of interest at the other end.

In a specific embodiment, the catheter presents an injection port, in particular implantable subcutaneously, at the end that can be connected to a source of delivery.

In a preferred embodiment, the kit further comprises a vessel adapted for containing or containing said compound of interest.

In a further embodiment, the kit further comprises a transfer element, in particular a pump, a needle, a syringe or a pen, making it possible to send the compound of interest from the vessel to the chamber within the catheter.

In a further embodiment, the kit further comprises sensors and/or captors to measure the level of a given biomarker in the blood and optionally send signals to the external source of the compound of interest. The biomarker is linked to the compound of interest, in the sense that its level is correlated with the need of the compound of interest for the patient. In case the biomarker levels go above or below preset levels, the sensors and/or captors shall send a signal to the external source, so that an adequate amount of the compound of interest is delivered through the device herein disclosed. In a particular embodiment, the biomarker is glucose and the compound of interest is insulin. The source of delivery is generally an implantable or an external vessel, optionally also comprising some transfer means such as a pump, a needle, a syringe or a pen, or an artificial organ. It may be part of the kit.

### Connector

In one embodiment, the implantable chamber comprises at least one connector comprising a body attached to the membrane, and a conduit connected to the connector so as to be in hydraulic communication with the inside of the pouch, which makes it possible to establish a communication between the exterior and the interior of the shell. This makes it possible to clip the catheter that will provide the substance of interest

US 2013216746 describes connectors that can be used with the chamber present in the kit. As for this patent application, a connector usable in the present kit may comprise a cap, a body and a base. The cap would comprise a central cavity connected to a sleeve, which receives the catheter, which is, for example, bonded to the inside of the sleeve. The body may have an annular shape and comprises three body teats projecting toward the cap. The cap may comprise three holes opposite the body teats so as to produce an assembly between the body and the cap by fitting the body teats into the holes. The body may also comprise an annular bulge corresponding to a recess of complementary shape produced in the cap, so as to clamp and hold one of the membranes forming the chamber, called an upper membrane, between the cap and the body.

WO2019011943 discloses an implantable chamber, having an inner space defined by two semi-permeable membranes, for diffusing at least one substance of interest, comprising an upper washer and a bottom washer configured to be oppositely placed on a side and on another side of two semi-permeable membranes, the upper and the bottom washers (120, 110) being tightly clipped together. Such washers can be used as connectors for the chambers herein disclosed.

In another embodiment, the chamber doesn't comprise connectors and the catheter (see below) is locked (integrated) between the two membranes forming the chambers, in particular in the weld joining the two membranes together.

### Catheter / setting of the catheter

The catheter used in the kit is any catheter known in the art, and is made in a biocompatible material. It is a flexible catheter which can be connected, at one end, to the connector on the surface of the chamber, so that the catheter's lumen is in relation with the interior volume of the chamber. The other end catheter may be connected to an injection port, a pump, or a vessel containing the compound of interest.

The internal diameter is generally in the range of 1 mm (i.e. it is generally between 0.5 mm and 1.5 mm, more preferably between 0.8 mm and 1.2 mm, or 0.9 mm and 1.1 mm). However, catheter of larger diameters may also be used.

The catheter is made in any biocompatible material such as silicone or any other biocompatible elastomer.

### Injection Port

Injection ports are known in the art. In the context of the present application, an injection port is a port that is to be placed near the surface of the skin of the patient (in a preferred embodiment, it is implanted subcutaneously).

In the context of the invention, an injection port may present a small chamber (portal, housing that defines a reservoir) with a septum disposed across an opening of the housing so as to cover the reservoir, wherein at least a part of this septum is penetrable by a needle. The septum is generally a rubber one, or can be made in silicone.

A needle can be used to inject the solution containing the compound of interest in the injection port, through the rubber septum. Once injected the solution will transitorily pass through the housing and flow to the implanted chamber by the catheter, where it will diffuse *in situ* through the pores of the membrane.

Implanting such an injection port makes it possible for the patient or the physician to easily inject the compound of interest while reducing the risks of infection and eliminating the pain associated with injections when multiple injections of the substance of interest is needed.

Multiple injection ports exist and have been described in the art, such as the the injection ports of the Districath® series (Districlass Medical SA, Saint-Etienne, France), the injection ports developed by B-Braun (Melsungen, Germany) or in US 20110184353.

One can use any other injection port such as the injection ports that are used in chemotherapy.

Because it is preferred when the port is implanted under the skin, it may be accessed using a dedicated needle or by connecting a pump through a catheter. The skin would be cleaned with an antiseptic and the needle inserted through the skin and the rubber septum. Ports can be implanted sub-cutaneous, on any appropriate location such as on the abdomen, but also on other areas such as the buttocks, thigh or arm.

The ports are generally made of titanium (or any thermoplastic such as Polysulfone) for their body (housing) and of silicone (septum).

The existing subcutaneous injection ports make it possible to perform multiple injections over the time. In particular, using the appropriate injection system, it may be possible to perform about 1000 injections of the compound of interest. For a patient with diabetes, which would make about 5 injections a day, this means that the ports could remain in place for about 200 days (8 months). This would ensure good healing, and prove to be an improvement in the life of patients who currently need to change the system every third days with external insulin pumps.

One can use any appropriate injection system such as a needle which would preferably be between 4 and 35 mm long, more preferably between 5 and 25 mm, most preferably between 8 and 13 mm. Alternatively, the needle may be between 20 and 32 mm. The diameter of the needle would preferably be between 18 and 32 gauges, most preferably between 22 and 32 gauges.

In another embodiment, the delivery device (chamber is linked to an external pump to an artificial pancreas (external pump + glucose reader/sensor) which delivers insulin when appropriate. In this embodiment, the duration of use of the device may be extended up to 4 to 5 years.

### Sensors

The kit herein described may also contain one or multiple sensors for monitoring glucose concentration in the blood of the host, and to provide measured data associated with the glucose concentration. An electronics module can also be part of the kit that will determine the insulin quantity to provide to the patient and send instructions to an insulin delivery device configured to deliver insulin to the host.

Such sensors are known in the art and are described in various publications such as US 9,463,277, US 9,457,146, US 9,452,260, US 9,452,259, US 9,452,258 or 9,283,323. This sensor is generally a continuous glucose monitoring (CGM), which will determine glucose levels at regular intervals (in the range of 2 to 5 minutes).

### Implantation of the chamber

The chamber of the kit, made with the membranes described herein which are flexible, is implanted at the most appropriate site to have a delivery of the compound of interest at a site of physiological interest.

As a matter of illustration, the chamber is implanted between muscles (abdominal muscles or rectus abdominis muscles) and the peritoneum when the compound of interest is insulin, for treating diabetes, in particular type 1 diabetes mellitus, or type 2 diabetes.

The chamber may be implanted, mini-invasively or not, at any site of interest, depending of the compound of interest to be delivered and the expected biodistribution. It can be implanted, in particular, sub-cutaneously, extraperitoneally, intra-abdominally, intraomentally, submucosally or intraperitoneally, more preferably extraperitoneally or submucosaly. The examples describe a specific procedure for extraperitoneal implantation.

In one embodiment, the device (chamber) can be implanted by making small incisions to introduce surgical instruments allowing to view the internal organs and placing the device in any abdominal cavity location suitable with the intended use.

In another embodiment, the technique used is the transabdominal preperitoneal (TAPP) procedure. Small incisions are also performed for the introduction of surgical instruments into the abdominal cavity. The peritoneum is opened to reach the preperitoneal space. After the placement of the device of the invention, the peritoneum is closed using sutures.

In another embodiment, small incisions are performed and surgical instruments and laparoscope are placed directly into the preperitoneal space without opening the peritoneum nor passing through the abdominal cavity. The layers are separated using for example a balloon filled with gas or liquid. This technique is also known as the totally extraperitoneal (TEP) procedure.

In another embodiment, laparotomy can be chosen. This technique aims to make a small incision near the navel is realized deepened through the skin, anterior rectus sheath, the rectus abdominis muscle, posterior rectus sheath until the peritoneum is exposed. A space is created above the parietal peritoneum layer where the device of the invention could be placed.

### Compound of interest

The compound/substance of interest is any compound/substance that is administered to a patient for treating the patient. It can be administered through a kit as disclosed or *via* cells encapsulated in the device.

### One can cite

Insulin for treating type 1 diabetes
A growth hormone for treating dwarfism
A coagulation factor (such as Factor VIII or IX) for treating hemophilia,
A cytokine or the like (tumor-necrosis factors, interferons...) or an anti-inflammatory molecule (whether nonsteroidal or steroidal) for treating auto-immune diseases such as arthritis, ankylosing spondylitis, multiple sclerosis, celiac disease, Graves disease, inflammatory bowel disease, psoriasis, rheumatoid arthritis, and systemic lupus erythematosus,
Heparin or heparinoids useful for treating coagulation,
A compound used in immunotherapy (such as a check-point inhibitor in particular PD-1 or PDL-1 inhibitors) for treating a cancer,
A drug used in chemotherapy (such as Paclitaxel, Taxol, Cis-Platin, cyclophosphamide, vincristine, 5-fluorouracile, or presnisolone) for treating a cancer.

An anti-coagulant factor to treat Hemophilia (such as Factor VIII or IX, or other factors).

A lysosomal enzyme (such as glucocerebrosidase, sphingomyelinase, beta-hexosaminidase A) for use thereof for lysosomal diseases
A pituitary hormone (such as growth hormone, adrenocorticotropic hormone, vasopressin, thyroid stimulating hormone (TSH), gonadotropic hormone) for use thereof for pituitary diseases.

An immunosuppressing (immunosuppressant) drug (such as a corticosteroid (in particular prednisone, budesonide, prednisolone), a Janus kinase inhibitor (in particular tofacitinib), a calcineurin inhibitor (in particular cyclosporine or tacrolimus), a mTOR inhibitor (in particular sirolimus or everolimus), a IMDH inhibitor (in particular azathioprine, leflunomide, mycophenolate), a biological product (in particular abatacept, adalimumab, anakinra, certolizumab, etanercept, golimumab, infliximab,ixekizumab, natalizumab, rituximab, secukinumab, tocilizumab, ustekinumab, vedolizumab, basiliximab, daclizumab) for treating cancer, auto-immune diseases, inflammatory diseases or Graft vs Host rejections,

An antiviral drug in particular adapted for health management and/or treatment of patients with HIV, herpes viruses, hepatitis B or C viruses (in particular ledipasvir/sofosbuvir, or sofosbuvir/velpatasvir), or influenza A and B viruses,
Arsenic for treating some auto-immune diseases,
TNF, useful for hepatitis C,
Dopamine for Parkinson disease treatment,
Eptifibatide (for reducing the risk of acute cardiac ischemic events and treating heart failure)
A beta blocker drug.
A pain management drug such as baclophene or bupivacaine.

One advantage of the kit herein disclosed is that it allows a diffusion of the compound of interest, an improved biodisponibility, and the possibility to easily ensure either continuous or discrete (but repeatable) administration of the compound of interest.

### Use of the kit

The kit as described herein may be used on human beings as well as on animals, in a veterinary usage, such as for insulin administration for dogs and cats.

The invention also pertains to a method for delivering a compound of interest to a patient comprising using a kit herein disclosed, in order to deliver the compound of interest to the interior of the chamber, from the external source, through the catheter. The invention also pertains to a method for treating a subject in need thereof, comprising administering, to a patient, a compound of interest useful for treatment of the patient to the interior of the chamber as herein disclosed, from an external source, through the catheter of a kit herein disclosed.

In these methods, the compound thus diffuses at the site of interest (site of implantation of the chamber). In a preferred embodiment, the site of interest is as described above.

In a specific embodiment, the chamber has previously been implanted within the subject's body via laparoscopic or NOTES (Natural Orifice Transluminal Endoscopic Surgery) procedure.

The invention also relates to a method of using the kit herein disclosed, comprising the step of surgically implanting the chamber within the subject's body, in particular by laparoscopic or NOTES (Natural Orifice Transluminal Endoscopic Surgery) procedure, in particular at a site as described above.

The invention also relates to insulin for its use for the treatment of diabetes wherein insulin is administered to the patient's body through a catheter linked to a chamber herein disclosed, and wherein insulin diffuses by the pores of the membranes of the chamber.

The invention also relates to heparin or heparinoids for use thereof for treating coagulation problems, wherein such compound is administered to the patient's body through a catheter linked to a chamber herein disclosed, and wherein the compound diffuses by the pores of the membranes of the chamber.

The invention also relates to a coagulation factor (such as Factor VIII or IX) for use thereof for treating hemophilia, wherein such coagulation factor is administered to the patient's body through a catheter linked to a chamber herein disclosed, and wherein the coagulation factor diffuses by the pores of the membranes of the chamber.

The invention also relates to a compound used in immunotherapy (such as a check-point inhibitor in particular PD-1 or PDL-1 inhibitors) for use thereof for treating a cancer, wherein such compound is administered to the patient's body through a catheter linked to a chamber herein disclosed, and wherein the compound diffuses by the pores of the membranes of the chamber.

The invention also relates to a chemotherapy drug (such as Paclitaxel, Taxol, Cis-Platin, cyclophosphamide, 5-fluorouracile, vincristine, or presnisolone) for use thereof for treating a cancer, wherein such drug is administered to the patient's body through a catheter linked to a chamber herein disclosed, and wherein the drug diffuses by the pores of the membranes of the chamber.

The invention also relates to a compound such as an immunosuppressing drug (such as a corticosteroid (in particular prednisone, budesonide, prednisolone), or to a Janus kinase inhibitor (in particular tofacitinib), or to a calcineurin inhibitor (in particular cyclosporine or tacrolimus), or to a mTOR inhibitor (in particular sirolimus or everolimus), or to a IMDH inhibitor (in particular azathioprine, leflunomide, mycophenolate), or to a biological product (in particular an antibody such as abatacept, adalimumab, anakinra, certolizumab, etanercept, golimumab, infliximab,ixekizumab, natalizumab, rituximab, secukinumab, tocilizumab, ustekinumab, vedolizumab, basiliximab, daclizumab) for use thereof for treating cancer, or an auto-immune disease, or an inflammatory diseases or Graft vs Host rejections, wherein the compound is administered to the patient's body through a catheter linked to a chamber herein disclosed, and wherein the compound diffuses by the pores of the membranes of the chamber.

The invention also relates to a lysosomal enzyme (such as glucocerebrosidase, sphingomyelinase, beta-hexosaminidase A) for use thereof for treatment of a lysosomal storage disease (LSD) such as Niemann-Pick disease, type C, Gaucher disease, such as Fabry disease and Hunter syndrome (MPS II), wherein such lysosomal enzyme is administered to the patient's body through a catheter linked to a chamber herein disclosed, and wherein the lysosomal enzyme diffuses by the pores of the membranes of the chamber.

The invention also relates to a pituitary hormone (such as growth hormone, adrenocorticotropic hormone, vasopressin, thyroid stimulating hormone (TSH), gonadotropic hormone) for use thereof treatment of a pituitary disease such as acromegaly, growth hormone deficiency, Sheehan syndrome, hypopituitarism wherein such pituitary hormone is administered to the patient's body through a catheter linked to a chamber herein disclosed, and wherein the lysosomal enzyme diffuses by the pores of the membranes of the chamber.

### Some Advantages of the kit

- Most compounds administered via the extraperitoneal or intraperitoneal route rapidly reaches the liver via the portal vein, more quickly than in the case of a subcutaneous administration. Diffusion of the compound by the chamber *in vivo* would be similar to direct intraperitoneal injection
- Using the kit would lead to an improved quality of life for patients
- A diffuse repartition of the compound, which will reduce or avoid any focal adverse effect (such as inflammation) or the like) linked to local over-concentration of the compound when administered
- There is no risk of infection (in comparison to the Diaport® system from Roche used for treating diabetes) since the system and the implantation procedure are safe
- No adhesion if extraperitoneal implantation, as compared with other devices implanted intraperitoneally (such as Diaport®) which may adhere to the tissues. This is particularly true if the surface of the chamber is coated with anti-inflammatory and/or anti-adhesion molecules.
- No risk of occlusion of catheters (in comparison to Diaport® system from Roche) by surrounding tissue, since the catheter is connected to the interior of the chamber.
- Long-time implantation when a sub-cutaneous injection port is used, when there is a need to change the catheter every three days for current insulin pumps.

### DESCRIPTION OF THE FIGURES

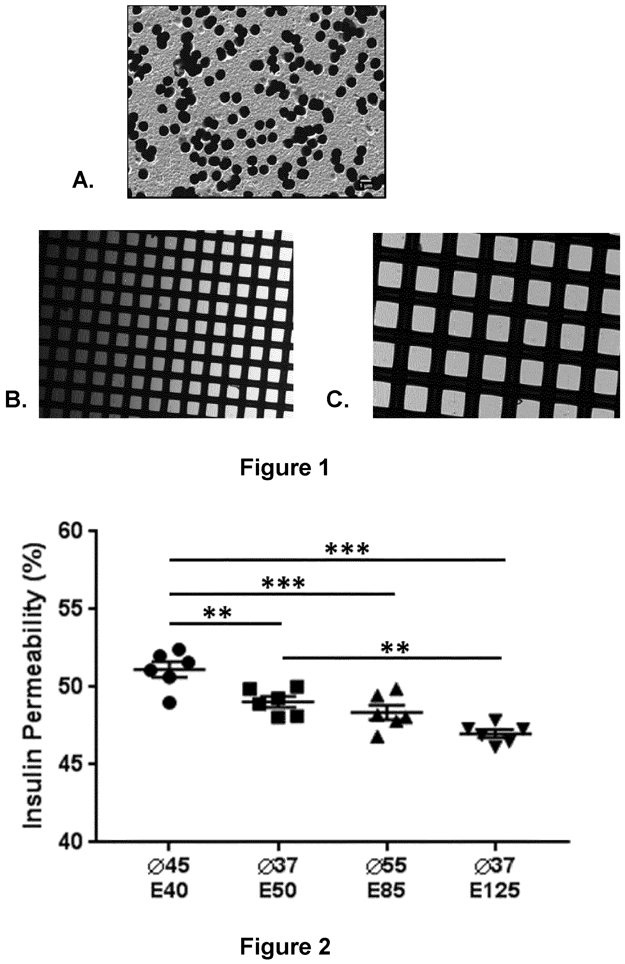

Figure 1: Comparison of pores repartition and homogeneity between membranes of the prior art (A) and membranes according to the invention (B, C). Scanning Electron Microscopy (SEM) of track-etched membranes (A) (The pores are in black and the membrane in light color), Optical microscopy of laser cut membrane with pores at 45µm, Magnificent x10 (B) and Laser cut membrane with pores at 45µm. Magnificent x20 (C). The pores are in light color and the membrane in black for B and C.
Figure 2: Permeability of Polyester membrane samples with different pore sizes (Ø, µm) and thicknesses (E, µm) to insulin. Diffusion time of 24h at 37°C, each point shows result of one sample, horizontal bar shows mean value with SEM as error bars. ** and *** p<0,01 and 0,001 respectively, One-way ANOVA with Tukey post-hoc test.

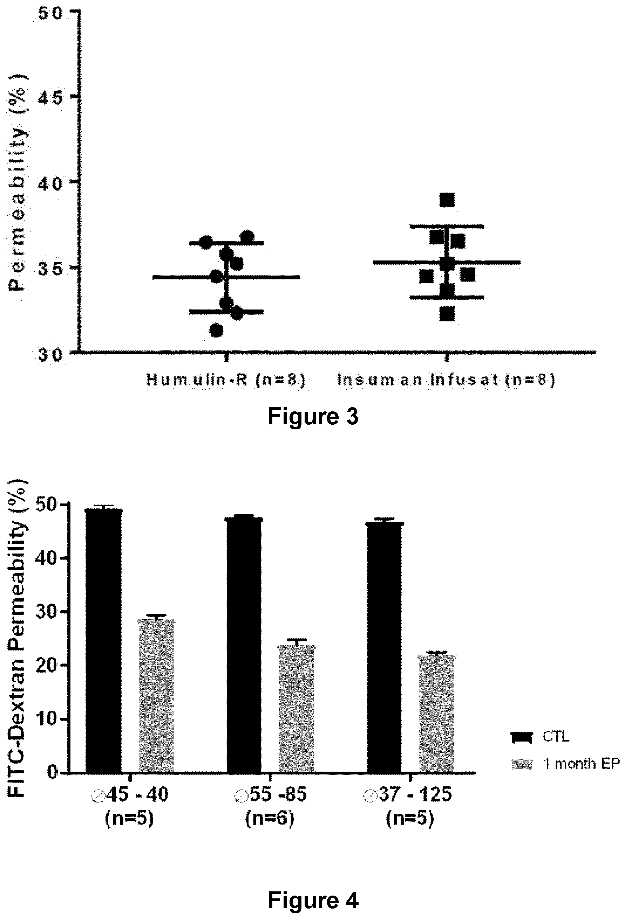

Figure 3: Permeability of symmetric membranes to Humulin-R® and Insuman lnfusat®after 24h. Each point shows a replicate, horizontal bar represents mean value and error bars corresponds to SD.
Figure 4: Permeability of membrane samples to 3-5 KDa FITC-Dextran after 4 weeks of implantation in extraperitoneal on diabetic rats compared to non-implanted samples. Mean±SEM. *** p<0,001 vs non implanted CTL, T.Test

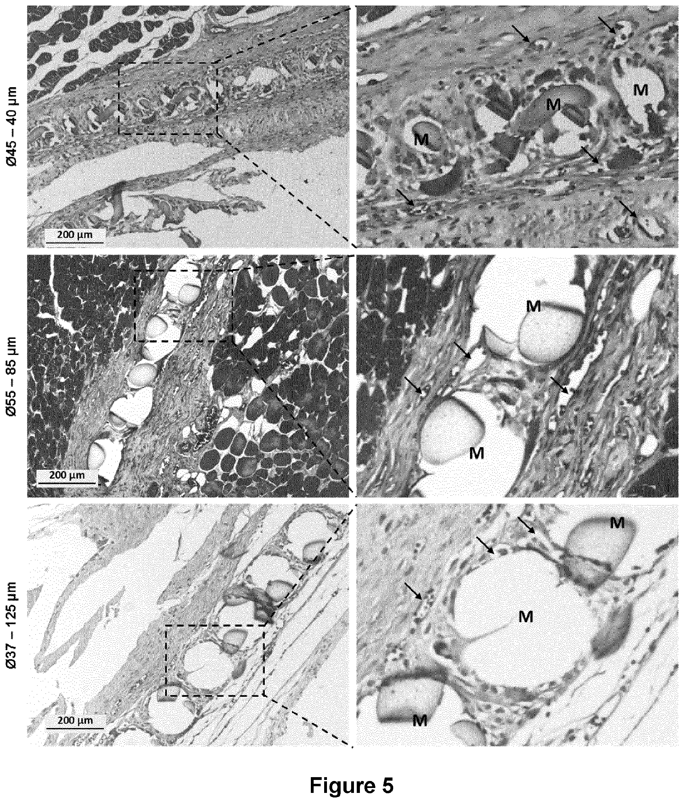

Figure 5: Masson's Trichrome staining reveals presence of vessels in direct contact with membrane candidates tested. Dotted square show area enlarged on right panel. Arrows indicate vessels in direct contact with membrane. M: membrane.

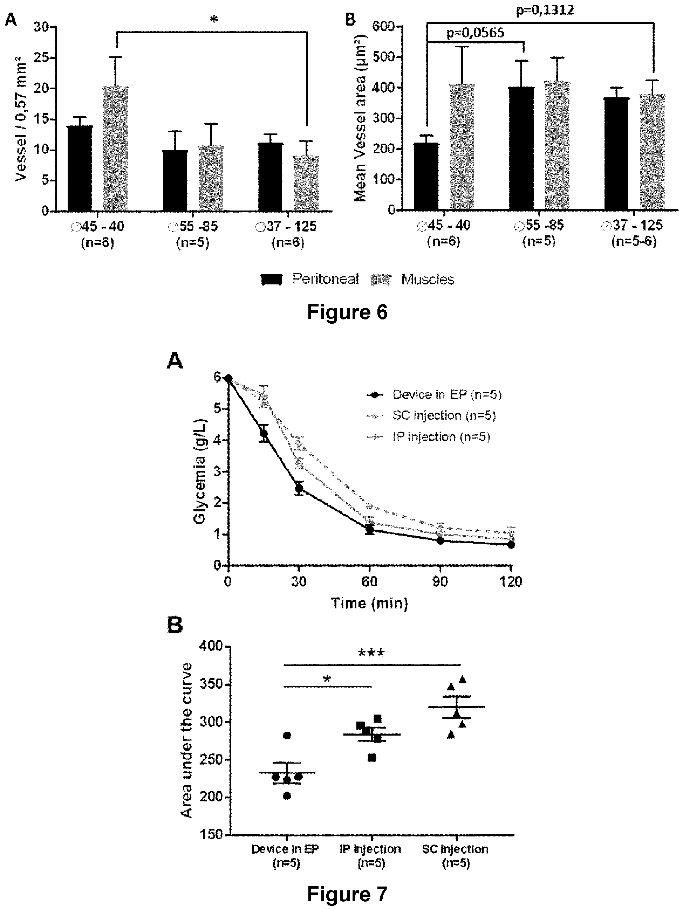

Figure 6: Number of vessels and their mean surface in tissues surrounding the three membrane candidates implanted for 4 weeks in extraperitoneal on rats. Mean±SEM. Quantifications were made separately on peritoneal and muscle side, on three non-overlapping field at magnification 10 of Masson's Trichrome stained tissue. * for p<0,05 one-way ANOVA with Tukey post-hoc test as data for peritoneal and muscle side were analyzed separately.
Figure 7: Glycemia follow up (A) and corresponding area under the curve (B) after injection of 2 IU of Human insulin in device made of laser cut membranes ExOlin® assembled with flexible membranes implanted in extraperitoneal or after direct IP or SC injection. Repeated measure ANOVA with Tukey Post-test. * For p<0,05 et ** for p<0,01

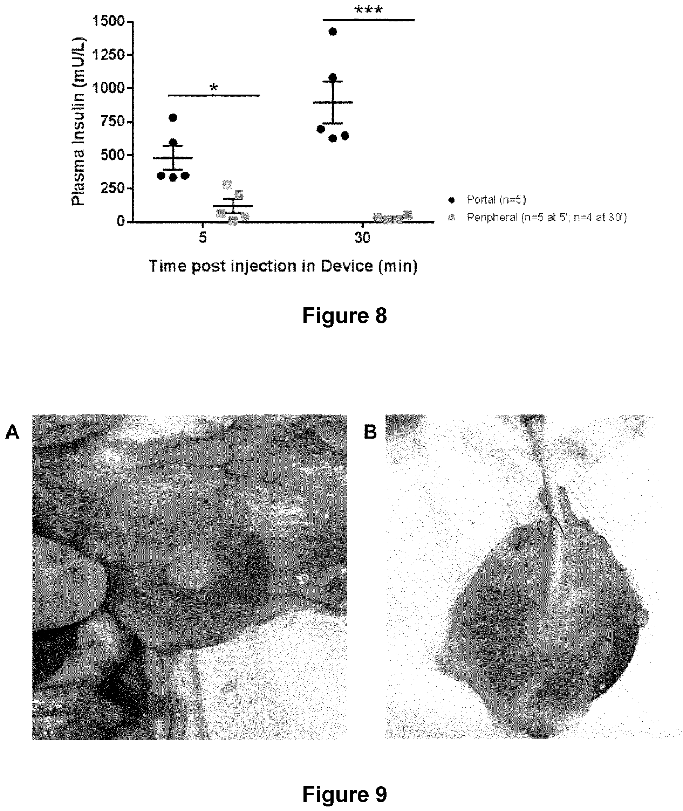

Figure 8: Insulin level in peripheral blood and portal vein at 5 min and 30 min after injection of 2IU of Human insulin in devices assembled with membranes implanted in extraperitoneal on diabetic rats.
Figure 9: Macroscopic view of new generation of ExOlin® adevice made with laser-cut membrane of the inventiont the time of retrieving after testing in diabetic rats (total implantation time: 7 weeks), on peritoneal side (A) and muscle side (B). Representative pictures of n=5.

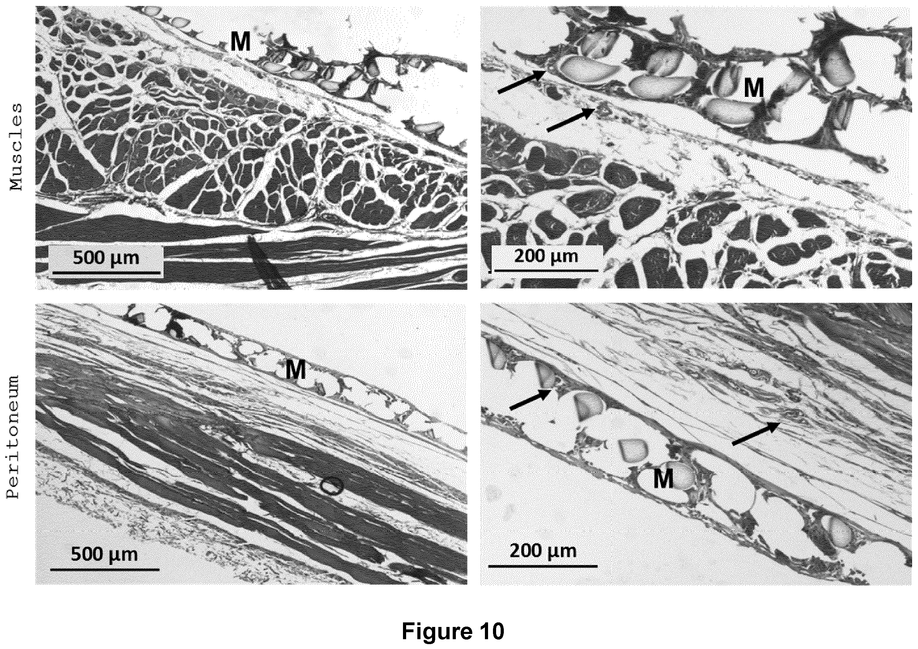

Figure 10: Masson's Trichrome staining of tissues surrounding laser-cut membranes at the time of retrieving the device after testing in diabetic rats (total implantation time: 7 weeks). Pictures of muscle side are presented on the upper panel and of peritoneum side on lower panel. Black arrow highlights vessels close to the membrane, M: place device's membrane. Representative pictures of n=5.

### EXAMPLES

### Example 1. Characterization of the membranes' surface in comparison to the prior art

Scanning Electron Microscopy (SEM) analysis shows a non-homogeneous distribution of the pores generated when using the track-etching process (as used in WO2015086550, WO2016184872 or WO2018087102). In comparison, the pores obtained using the laser technique are very reproducible and do not overlap thus increasing the repartition of the local constraints leading to a better mechanical properties (Figure 1).

It was also noticed that, in the conditions used, the pores obtained using the track-etching technique were smaller than the pores obtained with the laser cutting technique and were thus observed using SEM analysis, whereas the pores obtained with laser were observed using optical microscope.

### Example 2. Mechanical characterization of membranes according to the invention

The thickness of the 4 membranes that were tested varied from 40µm to 125µm (40µm, 50µm, 85µm and 125µm considered) with respectively 45µm, 37µm, 55µm and 37µm of pores diameters.

The test was performed using a tensile testing machine (MTS 1/M machine) equipped with a 500N load cell and a pneumatic clamping system adapted to the thinness of the membranes. No extensometer is available for this type of specimen (crosshead displacement taken in account). The strain rate was set to be 0,005s⁻¹.

All samples tested showed elongation at break comprised between 15 and 20 mm versus only 5 mm for the membrane of the prior art (obtained by track-etching). This reveals a high elasticity (and therefore flexibility) of the material tested.

It was noticed that the evolution of the maximum force is relatively linear as a function of the thickness of the membrane, except for the membrane of 85µm thickness because the structure of this membrane is different (larger pores) than the others. When comparing the membranes having 85µm and 125µm thicknesses (but different pore sizes), it was noted that the results were very similar considering either force or elongation (stress or strain).

From 85µm thickness, the membranes are stronger that those made of PET with track-etching. Indeed, the membranes are 4 times stronger than those from the prior art. Moreover, they can be more distorted (about 4 times more), but their stiffness is lower (lower Young's modulus). The membranes with a lower thickness (40µm and 50µm) underwent a lower force than those made of PET with a thickness 100µm, but they can be more distorted.

In view of these results, it was decided to further investigate the two membrane thicknesses (85 and 125 µm) for the development of a device suitable for mini-invasive surgery.

It was also noticed that mechanical resistance at break of the 1-layer laser cut membrane with homogenously distributed is at least as good as the 3-layers track-etched membrane from previous art.

### Example 3 Diffusion tests of molecules of interest through membranes

Discs of membranes were cut at a diameter of 22mm to fit the inner dimensions of a diffusion chamber developed to perform such testing. The diffusion chamber is composed of two compartments which can be screwed together using a PTFE O-ring to ensure the tightness of the chamber once assembled. The lower compartment is filled with a saline solution at 9g/L when the insulin diffusion is tested. Once the lower compartment is full, the 22mm disc of membrane is put in place and maintained by placing PTFE O-ring above. The upper compartment is then screwed and filled with the insulin solution to be tested. A lid is placed avoiding excessive evaporation.

The diffusion chamber is placed at 37°C during 24 hrs. Afterwards, a sample of medium in upper compartment is taken and the remaining is discarded. The upper compartment is then unscrewed and the disc of membrane is removed. A samples is collected also in the lower compartment and is assessed using BCA assay together with sample from upper compartment.

Then, permeability is calculated as the ratio of insulin found in lower compartment on total quantity of insulin in both upper and lower compartment. Since the testing is perform in static conditions, concentrations of upper and lower compartment tend to equilibrate if membrane is permeable to the molecule. Therefore, it is important to note that the maximal permeability that can be reached is 50%.

As first test with membranes displaying a thickness of 125µm and a pore diameter of 37µm showed high permeability to insulin. Three other membranes were tested. They displayed different pore diameter (Ø, µm) and thickness (E, µm) (Figure 2).

Results obtained after 24h diffusion test with insulin showed very high permeability of all the candidates. The highest permeability was obtained for membranes Ø45/E40 (51.13%), which is significantly higher than the three other candidates. This clearly highlights the decrease of insulin permeability with thicker samples. This trend is confirmed by the statistically higher permeability of membrane Ø37/T50 compared to the sample with same pore size but higher thickness of 125 µm (49.06±0.34% vs. 47.00±0.62%) (Figure 3). Despite significant differences between the four samples tested, permeability values are all higher than 40% and superior to those obtained with membranes from the prior art.

Results show that permeability of symmetric membranes to injectable insulin Insuman Infusat® (Sanofi) is comparable to permeability obtained with concentrated insulin Humulin-R® (Eli Lilly), used routinely for quality controls performed at reception of membranes. Permeability after 24h are respectively 34.4±2.0% and 35.3±2.1% for Humulin-R® and Insuman Infusat® (p=0.3923 Unpaired T.Test).

### Example 4: Testing of membranes' biointegration in rats

For each rat to implant, two discs of the same membrane (Ø45/E40, Ø55/E85 or Ø37/E125) were cut at a diameter of 22mm using a specific cutter. They were then placed in sealed pouch and sterilized with ethylene oxide.

Two discs were then implanted in extra-peritoneal site on healthy male Wistar rats (n=6 per membrane). One disc placed on the left side of the abdomen (for diffusion tests with 3-5 kDa FITC Dextran for 24h) and one on the right side (for histological and SEM analysis), to ensure having enough material to perform tests after retrieving. Implantation time was for 1 month.

Membrane discs implantation was performed following these steps:
- Make a 2 cm vertical incision using scalpel on the midline of abdomen
- Using fine scissors, separate skin from muscles
- On the side of linea alba, gently vertically incise the muscle with scalpel until peritoneum is exposed. Make sure incision is large enough to fit with membrane discs which have a 22 mm diameter.
- Dissect an extraperitoneal pouch using curved fine scissors and/or forceps. Be careful during dissection to avoid wound in the peritoneum.
- Once pouch is large enough, inject sterile saline solution and insert membrane disc in the pouch. Make sure disc is not folded on the site of implantation.
- Suture muscle using absorbable 4-0 thread (Vicryl Rapide - Ethicon), by sinusoid movement
- Suture skin using Donati pattern using absorbable 4-0 suture thread.

Animals implanted with membrane discs were kept for 4 weeks prior sacrifice by intraperitoneal injection of Pentobarbital (Euthasol® VET, 182 mg/mL) to retrieve membranes and surrounding tissues.

One of the two membrane disc was separated from surrounding tissues then rinsed in saline solution to perform diffusion test with 3-5 KDa FITC Dextran (size comparable to insulin) and determine if implantation period affected permeability of the sample. A small membrane samples was cut on the other disc for SEM analysis and the rest was taken with its surrounding tissues for histological analyses. Tissues with membranes were rinsed in saline solution then fixed in buffered formalin for 72h.

The three selected membrane candidates were implanted for 4 weeks in EP on non-diabetic rats, to investigate their biointegration, and permeability after implantation.

Membranes were macroscopically observable through peritoneum, at the time of sacrifice, revealing absence of strong fibrotic reactions. Membranes were not folded but difficult to separate from surrounding tissues, requiring to pull hard with forceps. Once separated form tissues, organic deposit stayed on the membrane and appeared to be pass through the pores.

These macroscopic observations were confirmed by SEM analysis that showed homogenous covering of membranes by organic deposit. The covering was identical for the three samples tested and specific surface patterns of membranes were hardly visible.

In addition to surface observation, permeability of membranes after 4 weeks of implantation in EP on rats was assessed with 3-5KDa FITC-Dextran. Choice of this molecule rather than insulin is explained by the presence of strong organic deposits that would interfere with BCA Assay validated for insulin titration.

In accordance with macroscopic observations and SEM analysis, results (Figure 4) follow the same trend for the three membrane candidates. A significant decrease (p>0,001 for all candidates) of permeability to 3-5 KDa FITC-Dextran is observed after 4 weeks of implantation compared to non-implanted material. Overall, permeability value on implanted membranes is half of the value obtained with non-implanted control (1.72-; 2.00- and 2.13-fold decrease for Ø45/E40; Ø37/E50; Ø55/E85 and Ø37/E125 respectively).

One of the two discs implanted for 4 weeks in EP on rats was not separated from its surrounding tissue and the whole sample was embedded in paraffin for histological analyses.

Regardless the membrane candidate implanted, they do not affect global tissue's organization both on muscular and peritoneal side, as shown by Hematoxylin-Eosin staining. Low cell infiltration was also observed together with very low fibrosis, indicating good acceptance of membrane in extraperitoneal. It is worth to mention that increased fibrotic tissue is observed on the edge of membrane discs, as observed with Masson's Trichrome especially for Ø45/E40 and Ø55/E85 candidates (right panel, upper and middle picture). In accordance with organic deposit found on membrane separated from tissues at retrieving, we observe a total enclosing of membrane in tissues. Tissue also passed through the pores, which explains the difficulties encountered to separate membranes and tissues.

Vascularization around membranes is a critical point. Therefore, this parameter was deeply analyzed.

Figure 5 shows pictures of vascularization in the area where membranes were implanted. In enlarged pictures from the left panel, one can observe small vessels that are very close to the membrane material (circular grey structures that seem to detach from the slide) as highlighted by black arrows. For Ø55/E85 and Ø37/E125 candidates, small vessels are observed directly attached on the membrane fibers and even present in some pores. For Ø45/E40 membrane, the vessels appeared to be more distant from the membranes and seem to be mainly located in fibrous tissue (stained in blue) close to the membrane material. These data could mean that the thickness of the membrane could have an impact on healing process as the distance between membrane and vessels is more important with the smallest thickness.

To have a more precise idea of vascularization tissue surrounding membrane candidates after 4 weeks of EP implantations, quantifications of vessel number and area was performed on slides stained with Masson's Trichrome staining.

Density of vessels is comparable on both peritoneal and muscle sides for membrane candidates Ø55/E85 and Ø37/E125. For Ø45/E40 membranes, density tends to be higher compared to the other candidates on peritoneal side (14.00±1.41 vs. 10.07±2.98 and 11.17±1.36 for Ø55/E85 and Ø37/E125 respectively).This trend is stronger on muscle side and vessels number is statistically higher with Ø45/E40 compared to Ø37/E125 candidate (p=0.0247, 20.44±4.75 compared to 9.11±2.32) (Figure 6).

Similar to vessels' density, mean vessel area is similar on both sides of the membrane, between Ø55/E85 and Ø37/E125. These mean values are also comparable to the one obtained with Ø45/E40 on muscle side. However, for this candidate, vessel area clearly tends to be lower than with Ø55/E85 and Ø37/E125 (220.2±24.7 for Ø45/E40 vs. 403.8±84.57 for Ø55/E85; p=0.0565 and 369.1±31.7 for Ø37/E125; p=0.1312). This reveals smaller vessels on peritoneal side for Ø45/E40 candidate compared to the two others.

Taken together, these data on vascularization show a higher number of vessels in tissues surrounding Ø45/E40 membrane with a smaller size. This points out that vascularization is less mature with this candidate compared to the others.

### Example 5: Efficacy study in diabetic rats of devices made from membranes

Since diffusion properties performed after the first implantation in rats were impacted with tissues deposition. Consequently, further implantations were realized with the whole device assembled with the same membranes on diabetic rats (induced by streptozotocin) to assess the effect of insulin despites tissue deposition. Diabetic rats under insulin therapy were implanted in EP (extraperitoneally) with device made of laser cut membranes with homogenously distributed pores (37µm pores and thickness of 125 µm). After a device pre-implantation period of 6 weeks, insulin therapy was stopped and injections of 2IU of insulin (Insuman Infusat®, 2IU) were performed. Each rat was successively injected with insulin in device made of laser cut membranes implanted in EP, in SC and in IP, with 48h washout period between each injection. After injection a 2h glycaemia follow up was performed with blood samples at 0, 30, 60 and 120 min to measure Human plasma insulin levels. Following these three injections, rats received a last injection of 2 IU of Human insulin in the device in EP and blood samples were taken at 5 and 30 min post injection in both portal and tail vein.

Finally, animals with the device made with laser-cut membranes were sacrificed to macroscopically observe its biointegration and integrity, and histological analyses were performed on tissues surrounding the device.

Figure 7A shows a faster glycemia decrease after injection of insulin in devices assembled with MEMBRANES, compared to direct injection of the same quantity of insulin in SC or IP. This results in a significantly lower AUC after injection through the device in EP compared to the two other conditions (p=0.0009 vs. SC injection; p=0.0204 vs. IP injection).

Results of Figure 8 clearly demonstrate that use of the device made of laser cut membranes in EP on diabetic rat model results in a rapid passage of insulin to portal vein, with significantly higher levels compared to peripheral blood at both times tested (p=0.0193 at 5 min and p<0.0001 at 30 min). This distribution pattern of injected insulin tends to mimic physiological situation and is comparable to what was observed with device made of membranes from the prior art.

At the end of the study, corresponding to a total time of 7 weeks of implantation, device did not show any folding and kept its integrity at the implantation site. Device was clean and significantly easier to retrieve compared to membranes alone. No thickening of peritoneum was observed and vessels were visible close to the membranes (Figure 9 A). On the muscle side aspect of tissue was also normal, without visible fibrotic reaction (Figure 9 B).

Histological analyses confirmed macroscopic observations, with very low fibrosis in contact with device's membrane on both muscle and peritoneal sides (Figure 10 left panel). In addition, vessels were observed on very close to the membranes and even in direct contact with membrane material (Figure 10 right panel).

Taken all together these results indicate that the device made with the laser-cut membranes is as efficient as previous version of the device. In addition, both material and topography of the membranes used provide a very good integration at extraperitoneal site in diabetic rats.

### Example 6: Laparoscopic procedure testing

Devices designed with laser cut membranes of 125µm with pores at 37µm were tested using laparoscopic tools in pigs. The diffusion chamber is inserted into the intraperitoneal space of the animal using a 12mm outer diameter trocar. Then the diffusion chamber was placed at the desired implantation site (i.e. submucosa, intra-abdominal). Diffusion chamber was also placed in the preperitoneal space by gently incising the peritoneum and suturing this latter when diffusion chamber was placed (transabdominal preperitoneal (TAPP) procedure).

In comparison with the diffusion chamber of the prior art, the chamber made with laser cut membranes bring real advantages for the minimally invasive surgery because of its flexibility and stiffness allowing respectively to pass through the 12mm diameter trocar but also unfold when in the intraperitoneal cavity without the need of additional tools, whereas the membranes previously described were impossible to roll to pass through trocar, without damaging them.

Moreover, the connection system of the chamber of the prior art was conserved and provides an easy grip for the grasper tool. In combination with the flexibility of the laser cut membranes, there is no need to develop additional apparatus, equipment nor tools to roll, inserted, unroll the diffusion chamber.

## Claims

1. A biocompatible implantable chamber, comprising a closed shell made of a membrane, delimiting an inner space, wherein the membrane comprises pores on at least one part of its surface, and wherein the pores are homogeneously distributed on the at least one part of the membrane.

2. The implantable chamber of claim 1, wherein the membrane comprises more than one a layer of biocompatible porous polymer.

3. The implantable chamber of claim 1, wherein the membrane consists in one layer of porous biocompatible polymer.

4. The implantable chamber of any one of claims 1 to 3, wherein the membrane comprising the homogeneously distributed pores is made from a polymer selected from the group consisting of polycarbonate (PC), polyurethane (PU), polyethylenimine, polyolefins (in particular polypropylene (PP) or polyethylene (PE)), polyester (in particular poly(ethylene terephthalate) (PET)), fluoropolymers (in particular polytetrafluoroethylene (PTFE) or polyvinylidene fluoride (PVDF)), and polyamides.

5. The implantable chamber of any one of claims 1 to 4, wherein the porous biocompatible polymer is made hydrophilic by surface physical or chemical modification, and covered with at least one hydrophilic polymer.

6. The implantable chamber of any one of claims 1 to 1, wherein the layer of porous biocompatible polymer has a pore density of between 10³ pores/cm² and 10⁹ pores/cm², preferably between 10⁴ pores/cm² and 10⁷ pores/cm², more preferably between 10⁴ pores/cm² and 10⁶ pores/cm².

7. The implantable chamber of any one of claims 1 to 6, wherein the total thickness of the membrane is between 5 µm and 250 µm, preferably between 5 µm and 200 µm, preferably between 10 µm and 150 µm.

8. The implantable chamber of any one of claims 1 to 7, wherein the porous biocompatible membrane is covered a hydrophilic polymer which contains at least one biologically active molecule, in particular covalently bound to a layer on the surface of said membrane.

9. The implantable chamber of any one of claims 1 to 8, wherein the pores have a diameter between 200 nm and 100 µm, preferably between 5 µm and 50 µm.

10. The implantable chamber of any one of claims 1 to 12, wherein it comprises at least one connector comprising a body attached to the membrane, and a conduit connected to the connector so as to be in hydraulic communication with the inside of the pouch, which makes it possible to establish a communication between the exterior and the interior of the shell.

11. A kit comprising
a. an implantable chamber according to any one of claims 1 to 10, which comprises at least one connector comprising a body attached to the membrane, and a conduit connected to the connector so as to be in hydraulic communication with the inside of the pouch
b. a catheter that can be connected to said connector at one end and that can be connected to a source of delivery of a compound of interest at the other end.

12. The kit of claim 11, wherein said catheter presents an injection port, in particular implantable subcutaneously, at the end that can be connected to a source of delivery.

13. The kit of claim 11 or 12, which further comprises a pump, a needle, a syringe or a pen, making it possible to send the compound of interest from the source of delivery to the chamber within the catheter.

14. The kit of any one of claims 11 to 13, further comprising sensors and/or captors to measure the level of a given biomarker in the blood and optionally send signals to the external source of the compound of interest.

15. Insulin for its use for the treatment of diabetes wherein insulin is administered to the patient's body through a catheter linked to a chamber according to any one of claims 1 to 10 and wherein insulin diffuses by the pores of the membranes of the chamber.
